# EUROPEAN PATENT SPECIFICATION

(11) **EP 0 647 432 B1**
(45) Date of publication and mention of the grant of the patent: **14.01.1998**
(21) Application number: 93307979.0
(22) Date of filing: 07.10.1993
(51) Int. Cl.: A61B 17/14

(54) **Instrumentation for preparing a distal femur**
Instrument zur Vorbereitung eines distalen Oberschenkelknochens
Instrumentation pour la préparation de l'extrémité distale d'un fémur

(43) Date of publication of application: 12.04.1995
(73) Proprietor: HOWMEDICA INC., New York New York 10017 (US)
(72) Inventor: Axelson, Stuart Lee, Jr., Lyndhurst, New Jersey 07071 (US)
(74) Representative: Ruddock, Keith Stephen

(56) References cited:
- EP-A- 0 502 738
- EP-A- 0 554 959

## Description

This invention relates to an apparatus for preparing a distal surface of a human femur. More particularly, it relates to an apparatus that can be used to prepare the surface of the femur to receive a distal femoral prosthesis.

Various types of instruments and methods have been developed to enable a surgeon to affix a distal femoral knee prosthesis to a human femur. Since the purpose for affixing such a prosthesis is to restore the patient's ability to walk after disease or other traumatic causes have impaired that ability, it is important that the prosthesis be attached to the femur in a manner that will approximate as closely as possible the natural condyles which the prosthesis is replacing.

It is a common practice to use the long central axis of the femur as a guide in determining the manner in which the distal femoral surfaces should be shaped to receive a properly aligned distal femoral prosthesis. Using the long central axis of the femur as a guide, a planar distal cut is first made on the distal femur and then the guide is removed and additional instrumentation utilizes the planar distal surface as a reference from which to cut anterior and posterior cuts, followed by anterior and posterior chamfer cuts.

Patents showing such a method or methods similar thereto are U.S. Patents 4,474,177 and 4,721,104. The former patent relates to a series of instruments for preparing the distal femur in which an angled alignment guide is used to index a plateau planar which is used to prepare the planar distal surface of the femur. The latter patent relates to a surgical apparatus for preparing the intracondylar area of the distal femur.

U.S. Patent 5,047,032 relates to a surgical instrument designed to produce a planar distal femoral surface. A router or side cutting drill is used to form the surface. Another patent which relates to the preparation of the distal femur is U.S. Patent 4,787,383, in which a saw is used to make the planar femoral cut. A saw guide is also disclosed. EP-A-0554959 discloses an apparatus for producing a planar surface on a portion of bone using an end milling device comprising a plate having an opening therein forming a guide means, means for securing the plate to the bone and a milling guide block coupled to the plate, said block having a reference surface and a track such that the milling device engages the reference surface to control the depth of cut and the milling device may be guided along the track in two dimensions to cut the bone a predetermined distance from the reference surface.

It is an object of this invention to provide an apparatus for forming planar surfaces on the media and lateral condyles of the distal femur which is simple in design.

The method for using the apparatus is simple to perform and therefore saves time during surgery.

The object is achieved by an apparatus for milling a planar distal femoral surface as defined in claim 1, which surface contains at least one of the medial and lateral condyles of a femur. The apparatus is initially located by use of an intramedullary rod extending into the intramedullary canal of the femur. The guide rod aligns a guide block which in turn aligns a ring-like femoral plate having a generally oblong opening therein which exposes both the medial and distal condyles when the plate is secured to the femur. The plate is secured to the medial and lateral surfaces of the distal femur by drill pins. The femoral bushing guide block fits within the generally oblong opening in the femoral plate and has a central hole therein angled laterally at an angle of about 8° from a direction perpendicular to the face of the femoral plate. Once the femoral plate is aligned, the guide block, bushing and rod are removed and a milling guide block having a pair of cylindrical medial and lateral guide bores therein adjacent the respective medial and lateral condyles is placed thereon. The milling guide block is inserted into the oblong opening in the femoral plate after the femoral plate is firmly secured to the femur and the femoral bushing and guide block which hold the intra-medullary rod is removed therefrom. The distal milling guide has a generally oblong shape which is snugly received within the oblong opening in the femoral plate. A cylindrical end mill cutter is inserted in at least one of the medial and lateral bores for milling said medal or lateral condyles. The end mill cutter has a bottom surface which could have at least two blades aligned to cut a flat surface. The milling of only one condyle would be required in a uni-condylar knee replacement wherein a prosthesis is implanted only on one of the condyles.

The milling guide block preferably has a circumferential rim thereon for engaging a flange on the distal end mill cutter to permit milling of the condyles to a predetermined depth. This depth can be varied by placing a ring-like spacer between the flange on the distal end mill cutter and the rim on the milling guide block which surrounds the opening in the milling guide block.

These and other objects and advantages of the present invention will become apparent from the following description of the accompanying drawings, which discloses one embodiment of the invention. It is to be understood that the drawings are to be used for the purposes of illustration only and not as a definition of the invention as defined by the claims.

In the drawings, wherein similar reference characters denote similar elements throughout the several views:
FIG. 1 is an exploded isometric view of the distal femoral plate of the apparatus of the present invention in combination with a bushing block and bushing for the alignment thereof;
FIG. 2 is an elevation view showing the distal femoral plate of FIG. 1 in alignment on and connected to the distal femur;
FIG. 3 is an exploded isometric view of the end mill and milling guide of the apparatus of the present invention prior to being inserted into the distal femoral plate;
FIG. 4 is an elevation view of the end mill of FIG. 3;
FIG. 5 is a bottom view of the cutting surface of the end mill of FIG. 4; and
FIG. 6 is an elevation view of the distal femoral plate mounted on a femur with the components of FIG. 3 located therein.

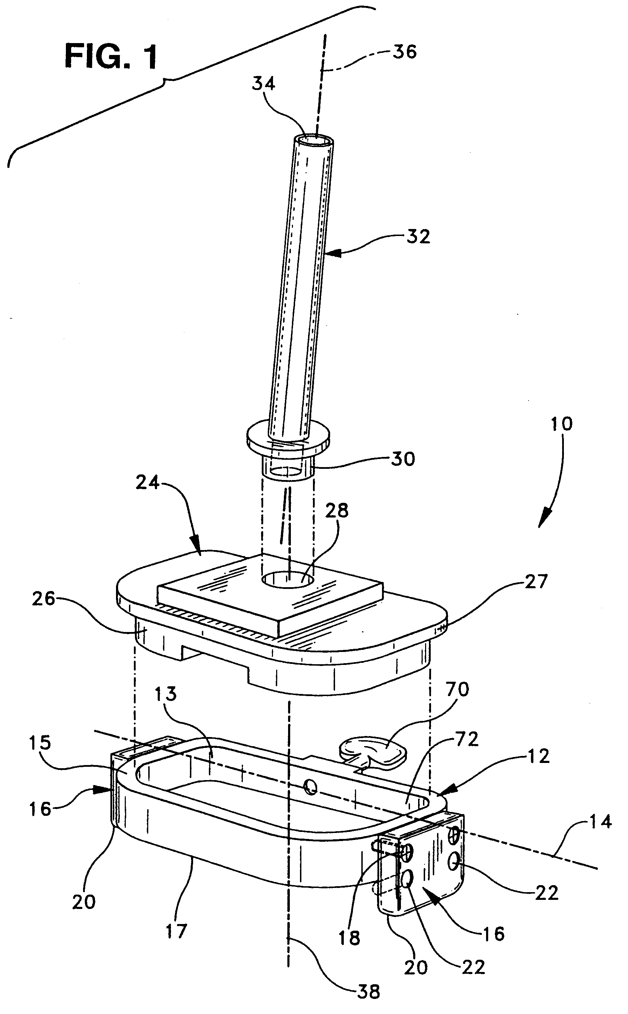

Referring to FIG. 1 there is shown a distal femoral plate 10 which, in the preferred embodiment, is in the form of an oval ring-shaped body 12 having a longitudinal axis 14. Body 12 has a top surface 15, a bottom surface 17 and an inner surface in the form of wall 13. Body 12 has a pair of flanges 16 at each end thereof which flanges are centered with respect to longitudinal axis 14 and, in the preferred embodiment, coupled to body 12 by screws 18.

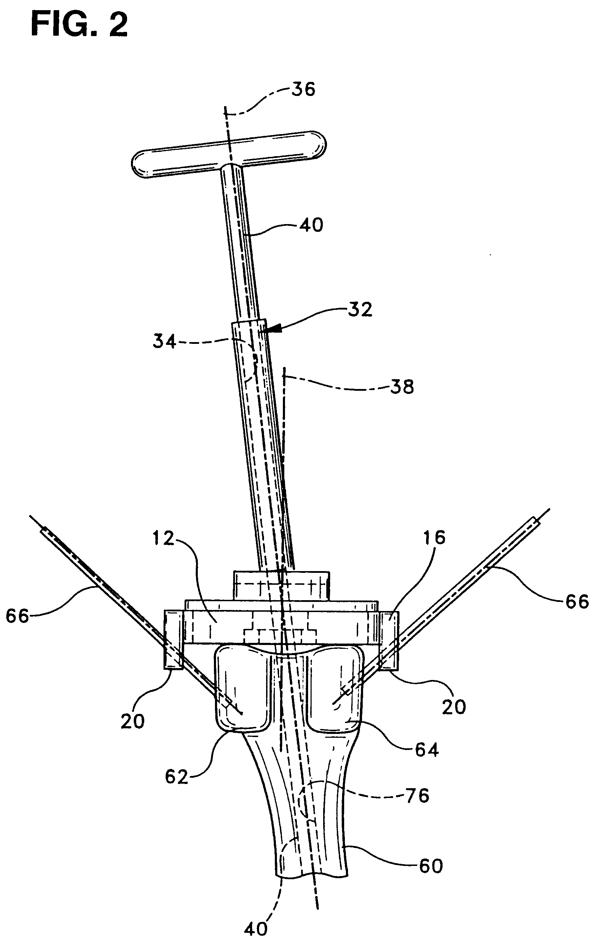

As seen in FIG. 2, the length of ring-shaped body 12 along longitudinal axis 14 in the preferred embodiment is selected to accommodate both the medial and lateral condyles 62, 64 of the distal femur 60. Thus, the distance between flanges 16 along axis 14 is sufficient so that the bottom surface 20 of each flange 16 extends proximally beyond the distal surface of the distal condyles. A pair of pin holes 22 are formed in flange 16 and are adapted to receive pins 66 which will be discussed in greater detail below. In the preferred embodiment holes 22 are angled inwardly toward the proximal end of femur 60. An axis 38 extending in a direction perpendicular to a plane containing top surface 15 of body 12 is located along axis 14 intermediate flanges 16.

Again referring to FIGS. 1 and 2, there is shown a femoral bushing guide block 24 having a flange 27 forming a stop surface thereon. A generally oblong wall 26 extends proximally from stop surface 27 of block 24. Wall 26 is shaped to be slidably received within interior surface 13 of ring 12. The lower surface of flange 27 engages top surface 15 of femoral plate 10 to limit the penetration of the guide block 24 therein. Femoral bushing guide block 24 also includes a central bore 28 co-axial with axis 38 and sized to receive cylindrical end 30 of an intramedullary rod bushing 32. Bushing 32 includes a bore 34 for receiving an intramedullary guide rod 40. Bore 34 in intramedullary rod bushing 32 extends along the longitudinal axis 36 which is angularly offset from bore 28 within femoral bushing guide block 26. In the preferred embodiment this offset is 5, 7 or 9°.

Preferably, intramedullary rod bushing 32 is made separate from femoral bushing guide blocks so that the angle between axis 36 and 38 may be varied, from 5° to 9° by a series of bushings 32, each having an axis 36 varying in 1 or 2° increments with respect to axis 38.

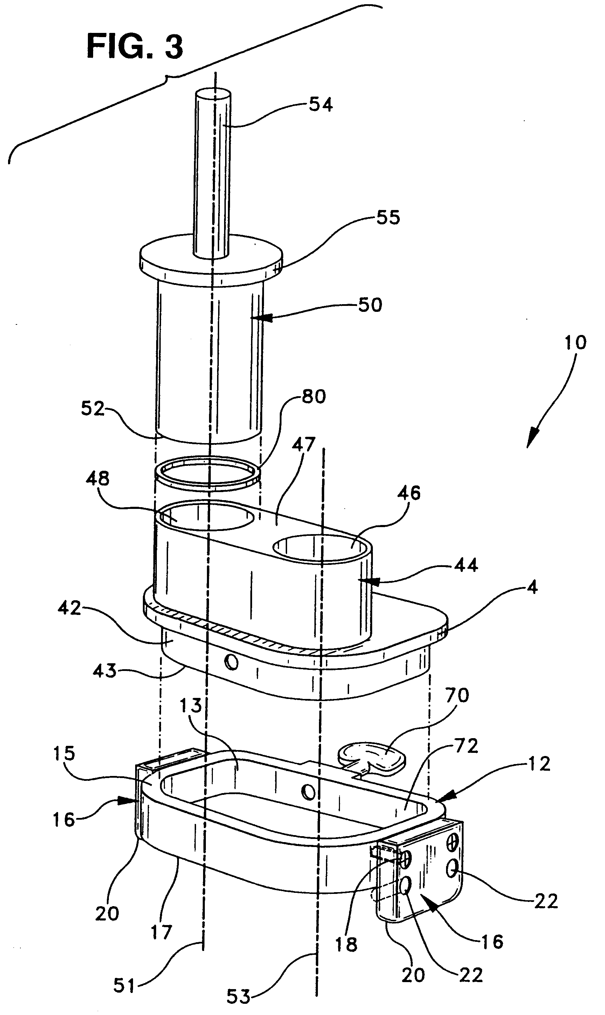

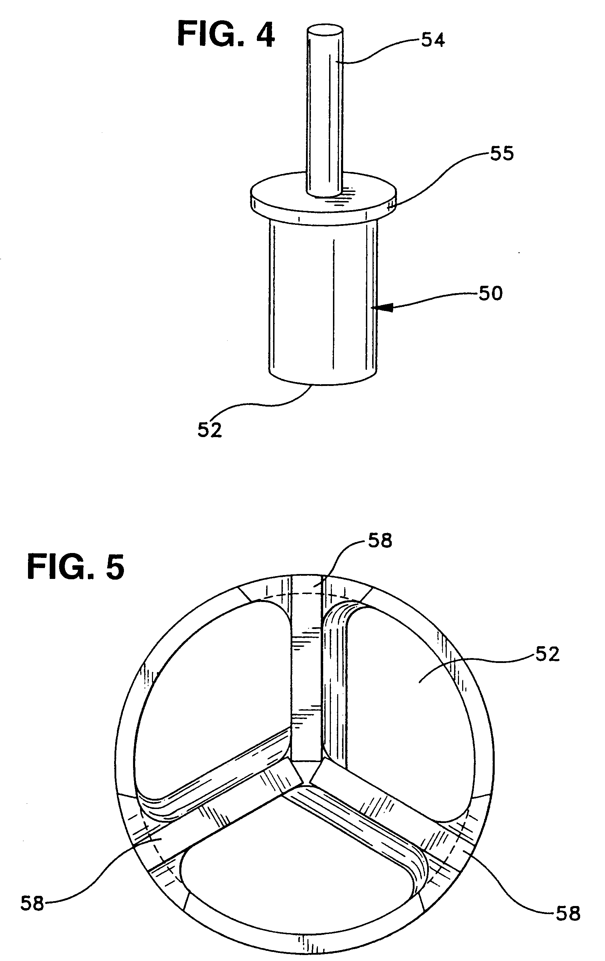

Referring to FIG. 3 there is shown a distal milling block 44 and femoral distal plate 10 as previously described. Milling guide 44 is designed to cooperate with the distal femoral plate 10 in a manner similar to bushing guide block 24. Surface 13 of ring 12 of plate 10 receives a proximally extending wall 42 of distal milling guide 44. Similar to guide block 24, milling guide 44 has a stop surface 41 which engages surface 15 of femoral plate 10. Distal milling guide 44 includes a pair of bores 46 and 48 respectively, which have longitudinal axes 51, 53 which are parallel with axis 38. In the preferred embodiment, the axes 51, 53 of bores 46 and 48 are offset with respect to a plane containing the axis 14 of distal femoral plate 10. The reason for this offset will be described in more detail below. The diameter of both bores 46 and 48 is sized to receive a distal end mill cutter 50 shown in FIGS. 3 and 4. End mill cutter 50 includes a milling surface 52 and a drive shaft 54 adapted to be connected to a suitable rotating power source. When inserted in either bore 46 or 48, the axis of drive shaft 54 is aligned with axes 51, 53. Spacers 80 may be provided to adjust the depth that surface 52 extends beyond the bottom surface 43 of wall 42 of guide 44 (i.e. to space flange 55 from surface 47 of guide 44).

Referring to FIGS. 4 and 5, it can be seen that surface 52 contains preferably three blades 58 for end milling a planar surface on the condyles of the distal femur. Two blades 58 or blades in excess of three may also be used.

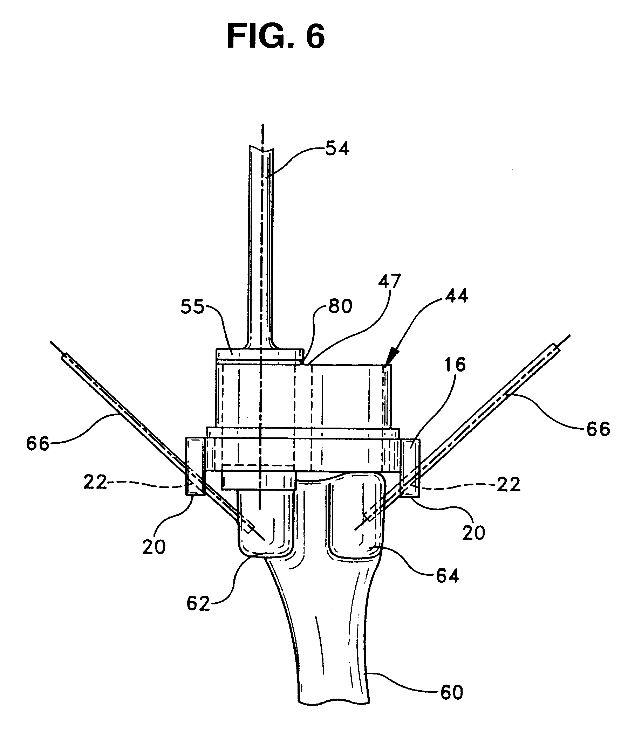

Referring to FIG. 6 there is shown the femoral plate of the apparatus of the present invention mounted on a distal femur 60 with mill cutter 50 in its final position. FIG. 6 shows distal femur 60 including condyles 62 and 64 with femoral plate 10 held thereon by four pins 66. Spacer 80 is shown in place on surface 47 of guide 44, which spacer 80 in the preferred embodiment, is one millimeter thick. One or more spacers may be used to adjust the depth of the condylar planar cut made by end mill 50.

The method of preparing a distal femoral surface by milling the medial lateral condyles 62, 64 of a femur 60 with the apparatus of the present invention will now be described. First a femoral bushing guide block 24 with an appropriately angled guide bushing 32 mounted in bore 28 thereof is placed into the distal femoral plate 10 with wall 26 slidably received with surface 13 of body 12. In the preferred embodiment a thumb screw 70 may be used to lock guide block 24 into femoral plate 10. In addition, the angular offset between axis 36 and 38 may be any angle between 5° and 9° with a series of bushings 32 being provided which result in an angular offset of 5, 7 or 9°.

After bushing 32, bushing guide block 24 and distal femoral plate 10 are assembled, intramedullary rod 40 is passed through bore 34 of the guide bushing 32 and into the medullary canal 76 of the femur 60. The assembly of distal femoral plate 10, guide bushing 24 and guide 32 are advanced distally on the rod 40 until contact is made with the prominent condyle 62, 64. Distal femoral plate 10 is then rotated so that a posterior wall 72 of surface 13 of plate 10 is parallel to a plane containing the posterior surface of the femoral condyles 62 and 64. Drill pins 66 are then placed through all four holes 22 of flange 16 and into the media-lateral sides of the condyles. In the preferred embodiment, holes 22 are angled toward the proximal end of femur 60 so that the pins 66 contact the medal and lateral sides of the condyles away from the distal area where the milling is to take place.

Once the femoral plate 10 is fixed in position by pins 66, the femoral bushing guide block 24, intramedullary rod guide bushing 32 and intramedullary rod 40 are removed. At this point in the surgery, the distal milling guide 44 is placed into the distal femoral plate and locked therein with the anteriorly positioned thumb screw 70. Distal end mill 50 is then inserted into circular bore 46 or 48 of milling guide 44. End mill 50 includes a flange 55 which contacts surface 47 of milling guide 44 and determines the depth of penetration of cutting surface 52 into the condyles 62 or 64. In the preferred embodiment, the milling guide penetrates the condyle distally 10 mm, at which time flange 55 contacts surface 47, thereby limiting penetration. Cylindrical spacer 80 may be used to limit the depth of penetration. For example, a 1 mm thick spacer 80 would limit the penetration of surface 52 into the condyles to 9 mm when it is inserted between flange 55 and surface 47 of guide 44.

The circular holes 46 and 48 are off center with respect to a plane containing a longitudinal axis 14 and perpendicular to the plane defined by the surfaces 15 of femoral plate 10 (i.e. they are offset in the anterior-posterior direction with respect to a medal-lateral plane through the femur). This allows the surgeon to flip the milling guide 180° about axis 38 and the longitudinal axis of the femur and mill additional bone in the anterior-posterior plane if necessary to prepare a larger planar surface on the distal femoral condyles. Because the circular bores 46 and 48 are offset in the anterior-posterior direction, a generally oval cut can be made on the condyles.

With the use of the system described above, extremely accurate flat bone cuts are made on both condyles 62 and 64. As with most knee instrumentation systems and surgical methods used for the preparation of the distal femur for implantation of a prosthetic femoral knee component, all other bone cuts are indexed off this first distal planar surface on the femur. It is contemplated that these other cuts are made in any well known manner after using the instrumentation of the present invention.

While one example of the present invention has been described, it is obvious that many changes and modifications may be made thereunto, without departing from the scope of the invention as defined in the claims hereafter.

## Claims

1. An apparatus for milling a distal femoral surface containing the media and lateral condyles of a femur (60) having an intramedullary canal (76), said apparatus comprising
a femoral plate (10) having a generally oblong opening (13) therein adapted for exposing both the media and distal condyles (62,64) when said plate is secured to the distal end of said femur;
means for securing said femoral plate (10) to said distal end ;
a milling guide block (44) coupled to said femoral plate, said guide block (44) having an outer surface (42) conforming to said generally oblong opening on said femoral plate for receipt therein and having a pair of cylindrical medial and lateral guide bores (46,48) therein for location adjacent the respective medal and lateral condyles; and
a cylindrical end mill cutter (50) for insertion in at least one of said medial and lateral bores in said guide block for milling at least one of said medial and lateral condyles.

2. The apparatus for milling a distal femoral surface as set forth in claim 1 wherein said milling guide block (44) has a stop surface (47) for engaging a radially extending guide surface (55) on said cylindrical end mill to permit the milling of said condyles to a predetermined depth.

3. The apparatus for milling a distal femoral surface as set forth in claim 2 further including at least one spacer (80) for insertion between said guide surface (55) on said end mill and said stop surface (47) on said milling guide block to vary said predetermined depth of said milling of said condyles.

4. The apparatus for milling a distal femoral surface as set forth in claim 1,2 or 3 wherein said medial and lateral guide bores (46,48) in said milling guide block are circular and are off center with respect to a plane containing the longitudinal axis (14) of said femoral plate such that when said block is used on said femur, said guide bores are offset in the anterior-posterior direction with respect to a mediallateral plane through the femur to allow for milling a generally oval planar surface on each condyle by flipping said block 180 degrees and thereby interchanging the guide bore adjacent each condyle used to make an initial cut.

## Patentansprüche

1. Vorrichtung zum Fräsen einer distalen femoralen Oberfläche enthaltend die medialen und lateralen Kondylen eines Femur (60) mit einem intramedullären Kanal (76), wobei die besagte Vorrichtung umfasst
eine femorale Platte (10) mit einer allgemein länglichen Öffnung (13) darin, die angepasst ist, um die beiden medialen und distalen Kondylen (62,64) freizulegen, wenn die besagte Platte am distalen Ende des besagten Femur befestigt ist;
Einrichtungen zum Befestigen der besagten femoralen Platte (10) an dem besagten distalen Ende;
einen mit der besagten femoralen Platte verbundenen Fräsführungsklotz (44), wobei der besagte Führungsklotz (44) eine der besagten allgemein länglichen Öffnung auf der besagten femoralen Platte entsprechende äußere Oberfläche (42) zur Aufnahme darin aufweist und ein Paar zylindrische mediale und laterale Führungsbohrungen (46,48) für eine Positionierung benachbart zu den jeweiligen medialen und lateralen Kondylen darin aufweist; und
einen zylindrischen Stirnfräser (50) zum Einführen in mindestens eine der besagten medialen und lateralen Bohrungen in dem besagten Führungsklotz zum Abfräsen mindestens eines der besagten medialen und lateralen Kondylen.

2. Vorrichtung zum Fräsen einer distalen femoralen Oberfläche nach Anspruch 1, bei welcher der besagte Fräsführungsklotz (44) eine Anschlagfläche (47) zum Eingriff mit einer radial verlaufenden Führungsfläche (55) auf dem besagten zylindrischen Stirnfräser aufweist, um das Abfräsen der besagten Kondylen bis zu einer vorbestimmten Tiefe zu ermöglichen.

3. Vorrichtung zum Fräsen einer distalen femoralen Oberfläche nach Anspruch 2, weiter enthaltend mindestens einen Abstandhalter (80) zum Einsetzen zwischen die besagte Führungsfläche (55) auf dem besagten Stirnfräser und die besagte Anschlagfläche (47) auf dem besagten Fräsführungsklotz, um die besagte vorbestimmte Tiefe des besagten Abfräsens der besagten Kondylen zu verändern.

4. Vorrichtung zum Fräsen einer distalen femoralen Oberfläche nach Anspruch 1, 2 oder 3, bei welcher die besagten medialen und lateralen Führungsbohrungen (46,48) in dem besagten Fräsführungsklotz kreisförmig sind und in Bezug zu einer die Längsachse (14) der besagten femoralen Platte enthaltenen Ebene außermittig sind, so dass bei Verwendung des besagten Klotzes auf dem besagten Femur die besagten Führungsbohrungen in Bezug zu einer Medial-Lateral-Ebene durch den Femur in der Anterior-Posterior-Richtung versetzt sind, um ein Fräsen einer allgemein ovalen planaren Oberfläche auf jedem Kondylus zu ermöglichen, indem man den besagten Klotz um 180 Grad dreht und dadurch benachbart zu jedem Kondylus die zum Herstellen eines ersten Schnitts verwendete Führungsbohrung austauscht.

## Revendications

1. Appareil servant à meuler une surface fémorale distale contenant les condyles médians et latéraux d'un fémur (60) ayant un canal intramédulaire (76), ledit appareil comprenant
une plaque fémorale (10) ayant en son sein une ouverture (13) globalement oblongue, adaptée de façon à exposer à la fois les condyles médian et distal (62, 64), lorsque ladite plaque est fixée à l'extrémité distale dudit fémur;
un moyen servant à fixer ladite plaque fémorale (10) à ladite extrémité distale;
un bloc de guidage de meulage (44) couplé à ladite plaque fémorale, ledit bloc de guidage (44) ayant une surface extérieure (42) se conformant à ladite ouverture de forme globalement oblongue se trouvant sur ladite plaque fémorale, afin d'y être logée et ayant en son sein un couple d'alésages de guidage médian et latéral (46, 48) cylindriques, en vue d'un positionnement de manière adjacente aux condyles médian et latéral respectifs; et
une lame de meulage d'extrémité cylindrique (50) destinée à être insérée dans au moins l'un desdits alésages médian et latéral dans ledit bloc de guidage afin de meuler au moins l'un desdits condyles médian et latéral.

2. Appareil servant à meuler une surface fémorale distale selon la revendication 1, dans lequel ledit bloc de guidage de meulage (44) présente une surface de butée (47) destinée à s'engager contre une surface de guidage (55) s'étendant radialement sur ladite lame de meulage d'extrémité cylindrique, afin de permettre le meulage desdits condyles sur une profondeur prédéterminée.

3. Appareil servant à meuler une surface fémorale distale selon la revendication 2, comprenant en outre au moins un élément d'espacement (80) destiné à être inséré entre ladite surface de guidage (55) sur ladite lame de meulage d'extrémité, et ladite surface de butée (47) sur ledit bloc de guidage de meulage, afin de faire varier ladite profondeur prédéterminée dudit meulage desdits condyles.

4. Appareil servant à meuler une surface fémorale distale selon la revendication 1, 2 ou 3, dans lequel lesdits alésages de guidage médian et latéral (46, 48) ménagés dans ledit bloc de guidage de meulage sont de forme circulaires et décalés par rapport à un plan contenant l'axe longitudinal (14) de ladite plaque fémorale de manière que, lorsque ledit bloc est utilisé sur ledit fémur, lesdits alésages de guidage soient décalés dans la direction avant - arrière, par rapport à un plan médian - latéral traversant le fémur, afin de permettre le meulage d'une surface planaire globalement ovale sur chaque condyle en faisant basculer ledit bloc de 180 degrés et en interchangeant de ce fait l'alésage de guidage adjacent à chaque condyle, utilisé afin de former une coupe initiale.
